# EUROPEAN PATENT SPECIFICATION

(11) **EP 2 437 740 B1**
(45) Date of publication and mention of the grant of the patent: **29.04.2015**
(21) Application number: 10716092.1
(22) Date of filing: 30.03.2010
(51) Int. Cl.: A61K 9/00, A61K 31/135

(54) **TREATMENT OF TINNITUS AND ASSOCIATED AUDITORY DYSFUNCTIONS**
BEHANDLUNG VON TINNITUS UND RELEVANTEN FUNKTIONSSTÖRUNGEN DES GEHÖRS
TRAITEMENT DE L'ACOUPHENE ET DES DYSFONTIONNEMENTS AUDITIFS ASSOCIES

(30) Priority: 31.03.2009 EP 09156913; 01.05.2009 US 174743 P
(43) Date of publication of application: 11.04.2012
(73) Proprietor: Tinnitus Research Initiative (TRI), 93053 Regensburg (DE)
(72) Inventor: COUTO DE BARROS COELHO, Claudia, 95860-000 Brasil (BR); RODRIGUES FIGUEIREDO, Ricardo, Volta Redonda/RJ (BR); DE NORA, Matteo, Principality of Monaco (MC); LANGGUTH, Berthold, 93049 Regenburg (DE); ELGOYHEN, Ana, Belén, Buenos Aires 1431 (AR)
(74) Representative: Cronin, Brian Harold John
(86) International application number: PCT/IB2010/051373
(87) International publication number: WO 2010/113109

(56) References cited:
- GB-A- 1 334 326
- US-A1- 2003 216 430
- US-A1- 2006 078 511
- US-B1- 6 632 843

## Description

### Field of the Invention

This invention relates to the treatment of tinnitus and related common auditory dysfunctions such as hyperacusis, auditory hallucinations, misophonia, phonophobia and central auditory processing disorders.

### Background of the Invention

### Tinnitus

Tinnitus is the phantom sensation of hearing in the absence of external sounds. Two main types can be identified: (1) objective tinnitus, which is caused by sounds generated somewhere in the body; (2) subjective tinnitus, which is the perception of meaningless sounds without any physical sound being present. Objective tinnitus is rare and is caused by a sound in the body, such as turbulent flow of blood or muscle contractions in the head. Such tinnitus can be heard by an observer in contrast to subjective tinnitus, which can only be heard by the individual who has the tinnitus. Subjective tinnitus is the most prevalent type of tinnitus. Tinnitus sounds can take a variety of forms such as buzzing, ringing, whistling, hissing or a range of other sounds. For some people it can even sound like music or singing. It can be a benign sound or it can prevent its sufferers from sleep or the ability to do intellectual work. All degrees of subjective tinnitus occur in between these extremes. Tinnitus is also related to other symptoms, such as hyperacusis, auditory hallucinations, misophonia, phonophobia and central auditory processing disorders. Affective disorders, such as anxiety and depression, often accompany severe tinnitus and that form of tinnitus can lead to suicide. Tinnitus is most likely related to altered neuronal activity which leads to plastic changes in the central auditory pathway derived from a distorted input. However, the mechanisms underlying the different forms of tinnitus remain incompletely understood.

Tinnitus often occurs as a result of dysfunction of the hearing system, such as from noise exposure, presbyacusis or from administration of specific pharmacologic agents. It can also be caused as the result of ear or head injuries, some diseases of the ear, ear infections and emotional stress. Perhaps the most common source of chronic tinnitus is exposure to loud sound. The noise causes permanent damage to the sound-sensitive cells of the cochlea, a spiral shaped organ in the inner ear. Carpenters, pilots, soldiers, rock musicians, street repair-workers are among those people whose jobs put them at risk. But also recreational use of sound, like MP3 players at maximal volume can produce damage. In addition, a long list of drugs can induce tinnitus. In some cases the causative agent remains unknown. One in 10 adults have clinically significant tinnitus (regular prolonged spontaneous tinnitus lasting 5 minutes or more), and for 1 in 100 adults tinnitus severely affects their ability to lead a normal life. Estimates indicate that 13 million people in western Europe and the USA currently seek medical advice for their tinnitus. Over 4 million prescriptions are written each year for tinnitus relief but these are all for off-label drugs from a wide variety of therapeutic classes and most are associated with considerable side effects. Despite the significant unmet clinical need for a safe and effective drug targeting tinnitus relief, there is currently no FDA-approved drug on the market that targets tinnitus.

Tinnitus can be associated with common auditory dysfunctions such as hyperacusis, distortion of sounds, misophonia, phonophobia and central auditory processing disorders. An extremely rare condition called "exploding head syndrome" has sometimes been called "explosive tinnitus"; however exploding head syndrome is not encompassed within the normal meaning of tinnitus or associated common auditory dysfunctions, and the present invention does not cover the treatment of the exploding head syndrome.

There are several therapeutic approaches to alleviate tinnitus, however, they have limited results and most patients are left unsatisfied. This includes: 1. counselling which can help the patient to cope with his tinnitus; 2. if tinnitus is accompanied by hearing loss, a hearing aid can help with tinnitus management; 3. sound therapy, also known as sound enrichment; 4. Tinnitus Retraining Therapy, a combination of counseling and sound therapy; 5. cognitive-behavioral therapy; 6. repetitive transcranial magnetic stimulation; 7. epidural cortical stimulation with implantable electrodes and 8. a series of off-label drugs like antidepressants, anxiolytics, anesthetics, anticonvulsants, analgesics, antiarrythmics, herbal medicines, anticoagulants, sedative-hypnotics, antihistaminergic compounds, antipsychotics, antioxidants, vasodilators, among others.

Specifically, the following proposals have been made in the patent literature, but have not been proven for widespread use:

US patent 4,735,968 discloses a method of treating tinnitus with aminoxyacetic acid (ADAA) administered orally. US patent 4,954,486 proposed treating tinnitus symptoms with furosemide. US patent 5,668,117 proposed treatment of tinnitus with a carbonyl trapping agent in combination with antidepressants or antianxiety medications; anticonvulsants; lidocaine; aminooxyacetic acid; praxilene; aniracetam; piracetam; 13-cisretionic acid; and 13-trans-retinoic acid. US patent 5,716,961 discloses the treatment of tinnitus using specific neuroprotective agents. US patent 5,863,927 proposed to treat tinnitus with dextromethorphan in combination with a debrisoquin hydroxylase inhibitor. US patent 6,358,540 disclosed the treatment of tinnitus with an herbal composition. US patents 6,656,172 and 6,969,383 proposed treatment of tinnitus using a catheter to infuse a therapeutic agent for example an agent comprising a local anesthetic such as lidocaine, or a GABA agonist. US patent 6,713,490 discloses a compound which is (R)-6[2-[4-(3-fluorophenyl)-4-hydroxy-1-piperidibyl]-1-hydroxyethyl]-3,4-dihydro-2(1H)-quinolinone *inter alia* for the treatment of tinnitus. US patent 6,770,661 disclosed various aryl substituted pyridines *inter alia* as antitinnitus agent.

### Cyclobenzaprine

Cyclobenzaprine is a skeletal muscle relaxant. The exact mechanism of action for cyclobenzaprine is unknown. Current research appears to indicate that cyclobenzaprine acts on the locus coeruleus where it results in increased norepinephrine release, potentially through the gamma fibers which innervate and inhibit the alpha motor neurons in the ventral horn of the spinal cord. Decreased firing of the alpha motor neuron results in decreased muscular tone. Cyclobenzaprine is a muscle relaxant acting primarily on the central nervous system. It is structurally similar to Amitriptyline, differing by only one double bond. Cyclobenzaprine is typically prescribed to relieve pain and muscle spasms. Typically, muscle spasms occur in an injury to stabilize the affected body part and prevent further damage. Whereas this is beneficial in acute injury, muscle spasm frequently persists over time, becomes dysfunctional and can increase the pain level. It is believed that by decreasing muscular spasm, pain is diminished. A common application would be that of a whiplash injury in a car accident. Cyclobenzaprine has also been studied in the treatment of fibromyalgia. In a study of 120 fibromyalgia patients, those receiving Cyclobenzaprine (10 to 40 mg) over a 12 week period had significantly improved quality of sleep and pain score. Interestingly, there was also a reduction in the total number of tender points and muscle tightness. It is also prescribed off-label as a sleeping-aid.

Cyclobenzaprine has been proposed in US patent 6,632,843 and patent application US 2006/06178511 for the treatment of bruxism by topical administration as a cream onto the skin overlying accessible muscles of mastication. Possible accessory alleviation of the other symptoms including tinnitus was also mentioned.

GB Patent Specification 1 334 326 proposes a composition having skeletal muscle relaxant activity comprising cyclobenzaprine and aspirin, which is intended to reduce the side effects of aspirin when administered in recommended dosages, such side effects including provoking epigastric distress, nausea, vomiting and tinnitus and more important, gastric erosion, exacerbation of peptic ulcer symptomes, perforation and haemorrhage.

Despite many suggestions for tinnitus treatment discussed above, there remains a need for an effective treatment of tinnitus that could be widely accepted.

### Summary of the Invention

According to one aspect, the invention provides cyclobenzaprine for use in the treatment of tinnitus and related auditory dysfunctions by oral administration or by parenteral administration through intramuscular, intravenous, subcutaneous or intrathecal injection or infusion.

Another aspect of the invention is the use of cyclobenzaprine in the manufacture of a medicament for the treatment of tinnitus and related auditory dysfunctions by oral administration or by parenteral administration through intramuscular, intravenous, subcutaneous or intrathecal injection or infusion.

Yet another aspect of the invention is a method of treating tinnitus and related auditory dysfunctions in a mammal, comprising orally administrating or parenterally administrating by intramuscular, intravenous, subcutaneous or intrathecal injection or infusion, to a mammal in need of such treatment, cyclobenzaprine in an amount sufficient to alleviate the symptoms of tinnitus or associated auditory dysfunctions.

In all of these aspects, the oral administration of about 10-80 mg/day of cyclobenzaprine to a subject suffering from tinnitus and related auditory dysfunctions provides alleviating affect, the most effective dosage being from 15-45 or from 20-40 mg/day, administered usually in doses 1-3 times per day. Excellent results have been achieved with a cyclobenzaprine dosage of 30mg/day. Using parenteral administration through intramuscular, intravenous, subcutaneous or intrathecal injection or infusion it may be possible to use a reduced dosage and better avoid side-effects.

The cyclobenzaprine is conveniently administered in tablet form but it can also be administered orally as capsules, as a powder or in solution and as a solution for injection or infusion. The administrated form can contain the usual pharmaceutically acceptable carriers, excepients or diluents.

Tablets or capsules can be designed for several times a day administration, or extended release for once-a-day administration.

The treatment according to the invention is in principle a long-term treatment extending over 2 weeks or more, or preferably over 4, 8 or even 12 weeks or more. In some patients positive effects were seen already after shorter treatment durations. In the below-described trials the treatment of tinnitus with cyclobenzaprine was weaned off after 12 weeks and tinnitus then got worse in some patients. It is noteworthy that the structure of the molecule cyclobenzaprine is similar to the structure of tricyclic antidepressants which have been shown to be safe for long-term administration. Moreover, cyclobenzaprine is on the market already for a long time, and its tolerability and side effects are well known.

As shown by the tests reported below, cyclobenzaprine has a positive effect on tinnitus severity and on tinnitus loudness in the tested subjects, it is safe to administer and though common side effects (like constipation and dry mouth) may be experienced, it is tolerated well by most subjects. Similar results are expected for associated auditory dysfunctions. Generally, according to the invention, cyclobenzaprine is effective for the treatment of an auditory dysfunction selected from tinnitus, hyperacusis, auditory hallucinations, misophonia, phonophobia and central auditory processing disorders.

Changes on tinnitus pitch were an unexpected effect of the tests, probably indicating that this medication induced a change on neural firing pattern. It was observed in 5 out of 15 subjects.

The time of tinnitus onset was not a predictive variable to positive outcome; subjects suffering for 28 years had positive results as well as subjects who had it for 8 months.

The duration of positive results is yet to be determined.

The invention will further be described by way of example in the tests reported below for tinnitus.

### Brief Description of the Drawings

Fig. 1 is a graph of the Global Improvement Scale of the tested subjects.
Fig. 2-6 are graphs showing the audiogram of different subjects; the progression of Minimum Masking Levels (MML) during the tests; and the progression of Tinnitus Handicap Inventory (THI) and Tinnitus Impairment Questionnaire (TBF12) during the tests.

### Description and Results of Trial Tests

From February to November 2008, 30 subjects were screened and 15 recruited to this trial. The sample counted on 7 males and 8 females with an age range from 36 to 71year, mean age 54.6 years (Std= 11.3 years).

### Medication

The selected subjects received Cyclobenzaprine tablets 3 times/day, as described below:

**Table 1**

| **Cyclobenzaprine dosage** | | | | | | | |
|---|---|---|---|---|---|---|---|
| | Baseline-week 2 | Week 2-4 | Week 4-8 | Week 8-12 | Week 12-13 | Week 13-14 | Week 14-16 |
| Concentration mg/day | 15 | 22.5 | 30 | 30 | 22.5 | 15 | 0 |

### Side effects:

The most frequent side effects among this sample were dry mouth and constipation. One subject had severe constipation and interrupted the study on week 8. Improvement on sleep and muscle relaxation was described by many patients.

### Difficulties during the trial

One subject didn't come to final tinnitus measurements. In another case, psychoacoustics measurements could not be performed because of the intermittent characteristics of tinnitus. The trial had to be interrupted in one case because of the medication's side effect although the subject was observing improvement on tinnitus.

### Results

Four selected outcome variables were used in this preliminary analysis: Global improvement (GI), Tinnitus Handicap Inventory (THI) and Tinnitus Impairment Questionnaire (TBF 12) questionnaires and Minimum Masking Levels (MML). The changes on the outcome variables were obtained by subtracting week 12's scores from the screening scores. The presence of a negative result means a decrease on values. On Subject number 6 the final scores correspond to week 8 (last observation carried forward analysis). Subject 3 had an intermittent tinnitus which interfered on the psychoacoustic examination.

**Table 2**

| **Changes on outcome variables ordered by Global Improvement (GI) classification.** | | | | | |
|---|---|---|---|---|---|
| **Subject number** | **Changes on** | | | | |
| | **GI** | **THI** | **TBF 12** | **MML** | |
| | | | | **Right** | **Left** |
| 2 | 1 | -14 | -2 | -5 | -8 |
| 3 | 1 | -30 | 6 | Not possible to measure but noticed change on intensity and less episodes during the day | |
| 8 | 2 | -48 | -11 | -11 | -17 |
| 4 | 2 | -8 | -3 | -25 | -12 |
| 10 | 2 | -14 | -2 | -8 | -9 |
| 14 | 2 | -10 | -10 | 2 | 0 |
| 11 | 2 | -28 | -5 | -5 | 1 |
| 13 | 2 | -40 | -7 | 1 | -8 |
| 9 | 3 | -14 | -1 | -7 | -7 |
| 5 | 3 | -42 | -4 | 0 | -4 |
| 6 | 3 | -38 | -4 | Trial suspended because of side effects | |
| 15 | 4 | -36 | -6 | Didn't return for measurements | |
| 7 | 4 | -32 | -3 | 9 | -7 |
| 12 | 4 | -28 | -6 | 3 | 8 |
| 1 | 4 | 0 | -3 | 14 | 3 |

In Table 2 the changes on outcome variables are listed by Global Improvement (GI) classification, as follows : GI 1 = much better; 2 = better; 3 = slightly better; 4 = no change; 5 = slightly worse; 6 = worse; 7 = much worse; THI: Tinnitus Handicap Inventory, TBF 12: Tinnitus Impairment Questionnaire; MML: minimal masking levels, measured in dB HL (Hearing Level).

Four different criteria were established to determine whether a subject responded or not to treatment (see Table 3). The prevalence of positive results to Cyclobenzaprine will reflect on how rigid the outcome criteria are selected.

**Table 3**

| **Criteria and prevalence of Cyclobenzaprine treatment results.** | | | |
|---|---|---|---|
| **Criteria** | **Responders % and n** | **Non responders % and n** | **Responders (subject #)** |
| **Criteria 1** | 73.3% | 26,7 % | 2, 3, 4, 5, 6, 8, 9, 10, 11, 13 and 14 |
| Improvement on GI | 11 | 4 | |
| **Criteria 2** | 73.3% | 26,4% | 2, 3, 4, 5, 6, 8, 9, 10, 11, 13 and 14 |
| Criteria 1+ decrease on THI | 11 | 4 | |
| **Criteria 3** | 66.6% | 33.3% | 2, 4, 5, 6, 8, 9, 10, 11, 13 and 14 |
| Criteria 2+ decrease on TBF 12 | 10 | 5 | |
| **Criteria 4** | 53.3 % | 46,7% | 2, 4, 5, 8, 9, 10, 11 and 13 |
| Criteria 3 + decrease on MML | 8 | 7 | |

Using the less conservative criteria, Criteria 1, based on subjects score to Global Improvement, (GI) 11 subjects (73.3%) referred that tinnitus improved with medication and 4 (26.7 %) didn't notice any change.

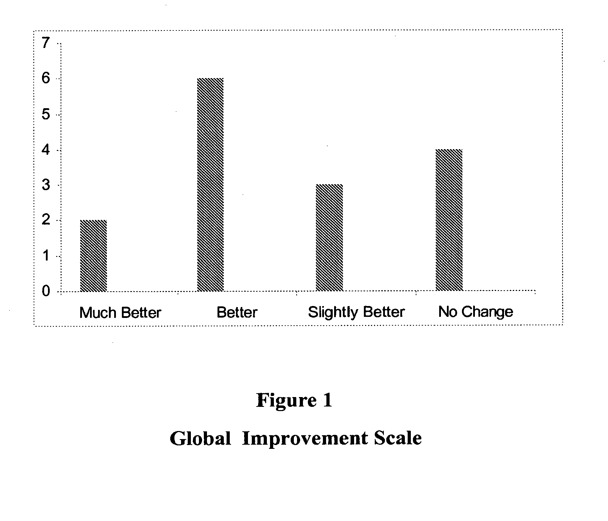

Different degrees of changes in GI perception are described on Figure 1. As can be seen, 8 subjects were Better or Much Better; and 11 subjects were Slightly Better, Better or Much Better.

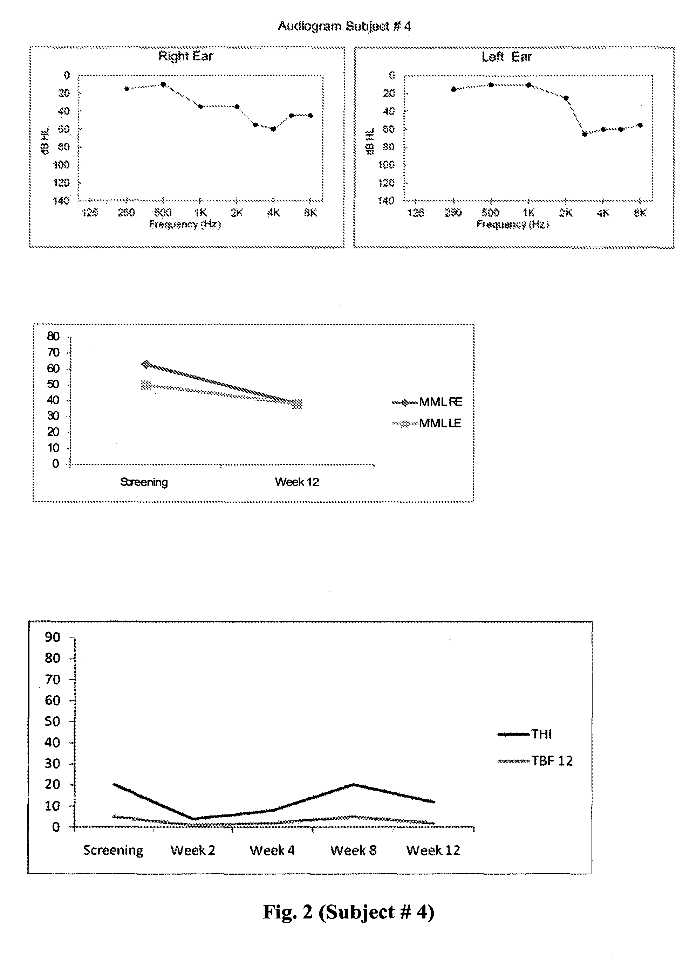

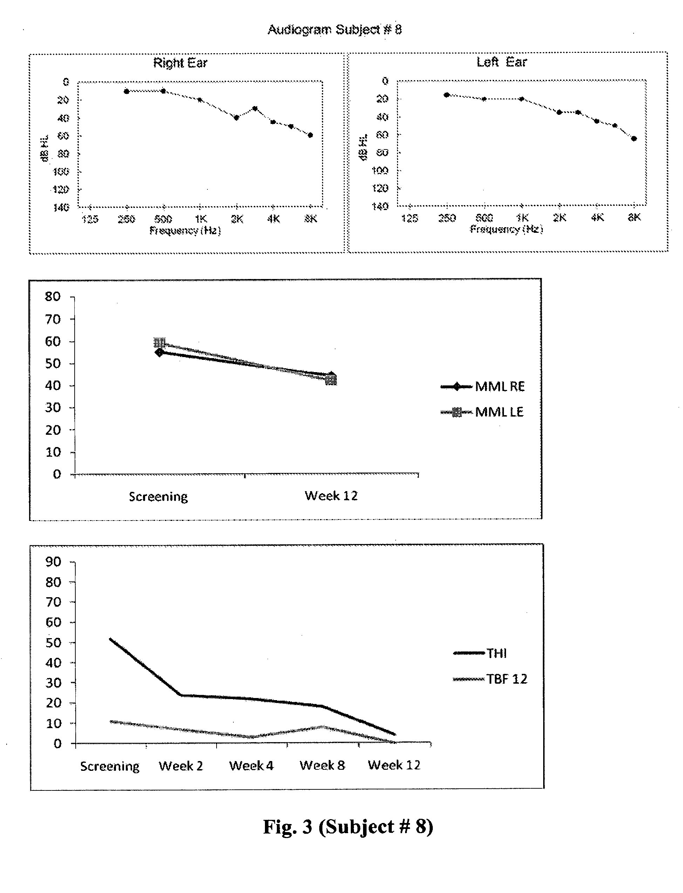

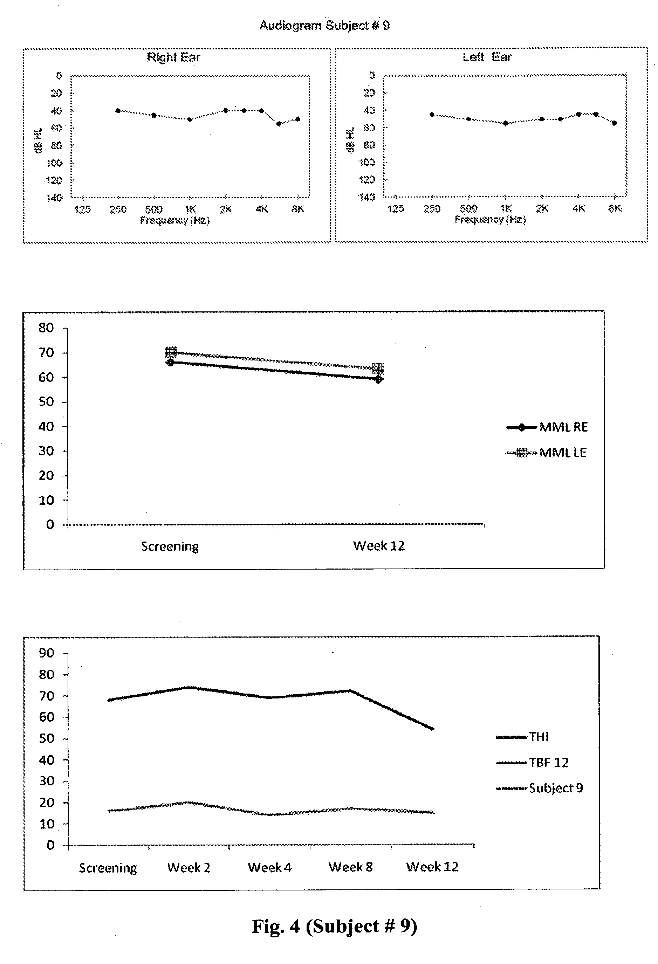

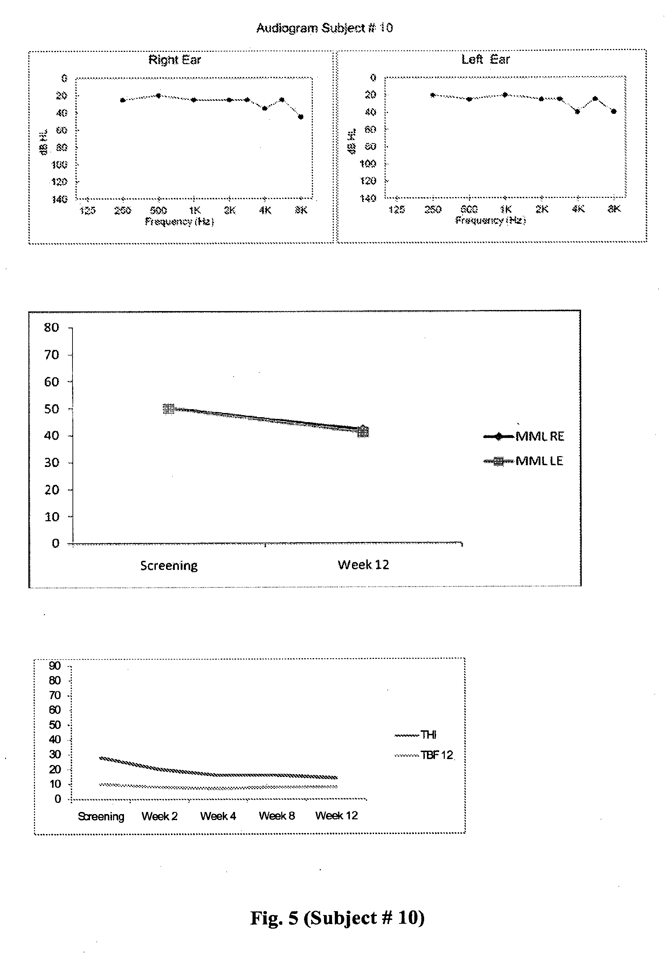

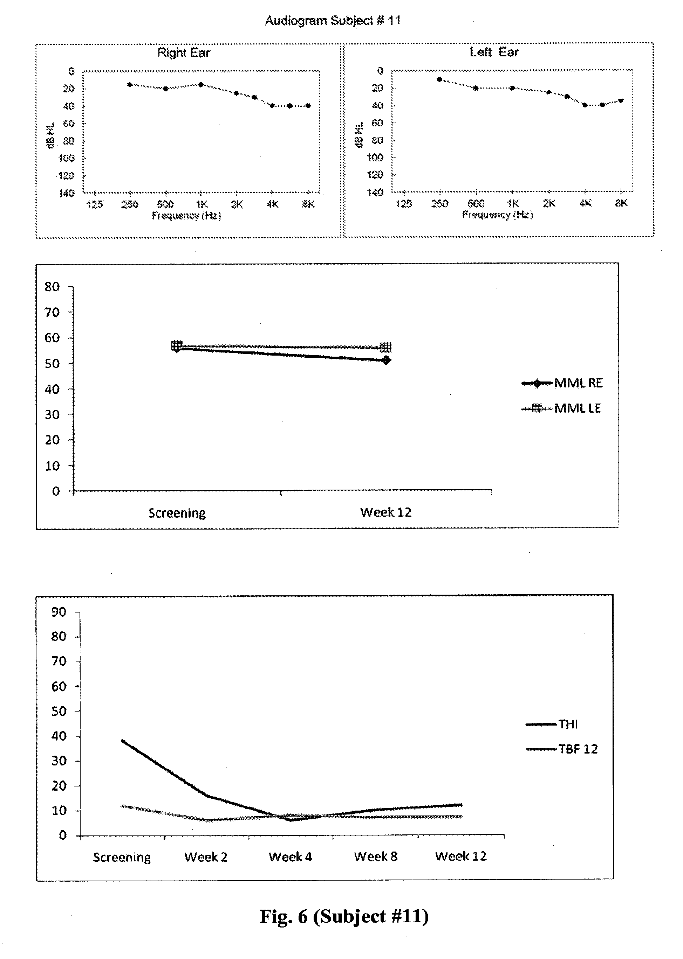

Figs 2-6 show by way of example results for subjects 4, 8, 9, 10 and 11 that responded to all of the Criteria 1-4. The progressive reduction of the Minimum Masking Levels (MML) in all of these subjects is particularly striking.

Using Criteria 4, the most conservative approach to select responders, only subjects that reported improvement on GI and demonstrated decrease on THI, TBF 12 and MML were included (see Table 2). Based on these criteria the rate of improvement was 53.3 %.

### General data on subjects and Tinnitus characteristics

Some of the data collected on this trial are listed on Tables 4 to 7, so one can have an idea about the bibliographical data, tinnitus characteristics and other variables collected on Case History Questionnaire. Tinnitus psychoacoustic measurements, audiograms as well as time line graphs for selected subjects showing THI and TB12 during different moments at the trial are presented in Figures 2 to 6.

**Table 4**

| **Case History Questionnaire Data of the 15 subjects** | | | | | | | | |
|---|---|---|---|---|---|---|---|---|
| **Subject number** | **Gender** | **Age** | **Family history** | **Etiology** | **Hearing** | **Time of onset (months)** | **Gradual/ sudden onset** | **Pulse** |
| 1 | M | 62 | Y | Other | Asymmetric NSHL* | 180 | Gradual | N |
| 2 | M | 43 | N | Other | Unilateral NSHL Left | 10 | Gradual | N |
| 3 | F | 57 | Y | Other | Unilateral NSHL Right | 180 | Sudden | YES, ≠ heart |
| 4 | M | 70 | Y | Noise | Asymmetric NSHL | 72 | Gradual | N |
| 5 | F | 61 | N | Stress | Asymmetric NSHL | 60 | Gradual | Y, Like heart |
| 6 | F | 36 | Y | Ear Infection | Normal | 8 | Gradual | Y, Like heart |
| 7 | M | 60 | N | Noise | Symmetric NSHL | 504 | Gradual | N |
| 8 | F | 71 | N | Other | Symmetric NSHL | 336 | Gradual | N |
| 9 | F | 57 | Y | Other | Asymmetric NSHL | 336 | Gradual | N |
| 10 | F | 69 | N | Other | Symmetric NSHL | 216 | Gradual | N |
| 11 | F | 43 | N | Hearing Loss | Symmetric NSHL | 24 | Sudden | N |
| 12 | F | 56 | N | Hearing loss | Asymmetric NSHL | 144 | Gradual | N |
| 13 | M | 40 | N | Noise | Symmetric NSHL | 216 | Gradual | Y |
| 14 | M | 45 | N | Noise | Asymmetric NSHL | 288 | Gradual | N |
| 15 | M | 49 | N | Barotrauma | Unilateral NSHL Left | 8 | Gradual | N |

| | | | | | | | | |
|---|---|---|---|---|---|---|---|---|
| * NSHL = neurosensory hearing loss | | | | | | | | |

**Table 5**

| **Tinnitus description from Case History Questionnaire** | | | | | | |
|---|---|---|---|---|---|---|
| **Subject number** | **Location** | **Constant/ intermittent** | **Changes On intensity** | **Intensity 0-100** | **Quality** | **Pitch** |
| 1 | BOTH EARS | Constant | Y | 30 | Crickets | High |
| 2 | LEFT EAR | Intermittent | Y | 50 | Wheezing | High |
| 3 | RIGHT EAR | Intermittent | Y | 70 | Crickets | Medium |
| 4 | BOTH EARS | Constant | Y | 30 | Crickets | High |
| 5 | BOTH EARS, WORST LEFT | Constant | Y | 70 | Wheezing | Very High |
| 6 | BOTH EARS, WORST LEFT | Constant | Y | 50 | Wheezing/ crickets | High |
| 7 | BOTH EARS | Constant | Y | 70 | Wheezing | Medium |
| 8 | LEFT EAR | Constant | N | 60 | Wheezing | Medium |
| 9 | BOTH EARS | Constant | Y | 80 | Tonal | High |
| 10 | BOTH, WORST RIGHT | Constant | N | 90 | Wheezing | High |
| 11 | BOTH, WORST RIGHT | Constant | N | 80 | Other | High |
| 12 | LEFT EAR | Intermittent | Y | 70 | Wheezing | Very High |
| 13 | BOTH EARS, WORST LEFT | Constant | N | 50 | Wheezing | Medium |
| 14 | BOTH, WORST RIGHT | Constant | N | 60 | Other | Very High |
| 15 | LEFT EAR | Constant | N | 70 | Crickets | High |

**Table 6**

| Associated Symptoms/ Somatic modulation | | | | | | |
|---|---|---|---|---|---|---|
| **Subject #** | **Headache** | **Vertigo** | **TMJ* Disfunction** | **Neck Pain** | **Other Pain** | **Somatic modulation** |
| 1 | N | N | N | Y | Y | N |
| 2 | N | N | N | Y | Y | N |
| 3 | N | N | N | Y | N | N |
| 4 | Y | Y | N | Y | Y | Y |
| 5 | N | N | N | Y | Y | N |
| 6 | Y | Y | Y | Y | Y | Y |
| 7 | Y | Y | Y | Y | Y | N |
| 8 | N | N | Y | Y | Y | N |
| 9 | Y | Y | Y | N | Y | N |
| 10 | N | N | N | N | Y | N |
| 11 | Y | Y | Y | Y | Y | N |
| 12 | Y | N | N | N | N | Y |
| 13 | Y | Y | N | Y | N | N |
| 14 | N | N | Y | Y | N | Y |
| 15 | N | N | Y | Y | Y | Y |

| | | | | | | |
|---|---|---|---|---|---|---|
| * Temporomandibular Joint Dysfunction | | | | | | |

**Table 7**

| **Tinnitus Psychoacoustic characteristics collected at screening and at week 12 ("12W")** | | | | | | | | |
|---|---|---|---|---|---|---|---|---|
| | **Pitch (kHz)** | | **MML Right Ear (dB HL)** | | **MML Left Ear (dB HL)** | | **Residual Inhibition** | |
| **Subject #** | **Screening** | **12 W** | **Screening** | **12 W** | **Screening** | **12 W** | **Screening** | **12 W** |
| 1 | 2 | 3 | 38 | 52 | 42 | 45 | Complete | No |
| 2 | Broad Band | Broad Band | 50 | 45 | 70 | 62 | Complete | Complete |
| 3 | --- | 6 | -- | -- | -- | --- | --- | --- |
| 4 | 8 | 4 | 63 | 38 | 50 | 38 | Complete | Partial |
| 5 | 4 | 4 | 53 | 53 | 49 | 45 | Complete | Complete |
| 6 | Broad Band | -- | 15 | | 24 | | No | - |
| 7 | 2 | 2 | 35 | 44 | 44 | 37 | Partial | Partial |
| 8 | 6 | Broadband | 55 | 44 | 59 | 42 | Partial | Complete |
| 9 | 4 | 6 AND 1 | 66 | 59 | 70 | 63 | Complete | Complete |
| 10 | 8 | Broadband | 50 | 42 | 50 | 41 | Complete | Complete |
| 11 | 8 | 4 | 56 | 51 | 57 | 56 | Partial | Partial |
| 12 | Broadband +2K | Broadband +2K | 40 | 43 | 37 | 45 | Complete | Complete |
| 13 | 8 | 8 | 15 | 16 | 25 | 17 | Complete | Complete |
| 14 | 4 | 4 | 35 | 37 | 34 | 34 | Partial | Partial |
| 15 | 6 | | 40 | | 57 | | Complete | --- |

## Claims

1. Cyclobenzaprine for use in the treatment of tinnitus and related auditory dysfunctions by oral administration or by parenteral administration through intramuscular, intravenous, subcutaneous or intrathecal injection or infusion.

2. Cyclobenzaprine for use according to claim 1 in a form for administration to a subject suffering from tinnitus or related auditory dysfunctions in an amount of 10-80 mg/day.

3. Cyclobenzaprine for use according to claim 2 for administration in an amount of 15-45 or 20-40 mg/day.

4. Cyclobenzaprine for use according to claim 1, 2 or 3 in the form of tablets, capsules, powder or solution.

5. Cyclobenzaprine for use according to any preceding claim for a long-term treatment of tinnitus and related auditory dysfunctions extending over 2 weeks or more, preferably 8 weeks or more.

6. Cyclobenzaprine according to any preceding claim for use in the treatment of an auditory dysfunction selected from tinnitus, hyperacusis, auditory hallucinations, misophonia, phonophobia, and central auditory processing disorders.

## Patentansprüche

1. Cyclobenzapyren zur Verwendung in der Behandlung von Tinnitus und verwandten Hörstörungen durch orale Verabreichung oder durch parenterale Verabreichung durch intramuskuläre, intravenöse, subkutane oder intrathekale Injektion oder Infusion.

2. Cyclobenzapyren zur Verwendung gemäß Anspruch 1 in einer Form zur Verabreichung für ein unter Tinnitus oder verwandten Hörstörungen leidendes Subjekt, in einer Menge von 10-80 mg/Tag.

3. Cyclobenzapyren zur Verwendung gemäß Anspruch 2 zur Verabreichung in einer Menge von 15-45 oder 20-40 mg/Tag.

4. Cyclobenzapyren zur Verwendung gemäß Anspruch 1, 2 oder 3 in der Form als Tabletten, Kapseln, Pulver oder Lösung.

5. Cyclobenzapyren zur Verwendung gemäß eines der vorstehenden Ansprüche zur langfristigen Anwendung von Tinnitus und verwandten Hörstörungen über 2 Wochen oder mehr, bevorzugt 8 Wochen oder mehr.

6. Cyclobenzapyren gemäß eines der vorstehenden Ansprüche zur Verwendung in der Behandlung einer Hörstörung ausgewählt aus Tinnitus, Hyperakusis, akustische Halluzinationen, Misophonie, Phonophobie, und zentralen auditiven Verarbeitungsstörungen.

## Revendications

1. Cyclobenzaprine pour une utilisation dans le traitement des acouphènes et des dysfonctionnements auditifs apparentés par administration orale ou par administration parentérale via une injection ou une perfusion intramusculaire, intraveineuse, sous-cutanée ou intrathécale.

2. Cyclobenzaprine pour une utilisation selon la revendication 1 sous une forme pour administration à un sujet souffrant d'acouphènes ou de dysfonctionnements auditifs apparentés en une quantité de 10-80 mg/jour.

3. Cyclobenzaprine pour une utilisation selon la revendication 2 pour administration en une quantité de 15-45 ou 20-40 mg/jour.

4. Cyclobenzaprine pour une utilisation selon la revendication 1, 2 ou 3 sous forme de comprimés, gélules, poudre ou solution.

5. Cyclobenzaprine pour une utilisation selon l'une quelconque des revendications précédentes pour un traitement à long terme des acouphènes et des dysfonctionnements auditifs apparentés s'étendant sur 2 semaines ou plus, de préférence 8 semaines ou plus.

6. Cyclobenzaprine selon l'une quelconque des revendications précédentes pour une utilisation dans le traitement d'un dysfonctionnement auditif sélectionné parmi les acouphènes, l'hyperacousie, les hallucinations auditives, la misophonie, la phonophobie et les troubles du traitement auditif central.
